# EUROPEAN PATENT APPLICATION

(11) **EP 4 269 558 A1**
(43) Date of publication of application: **01.11.2023**
(21) Application number: 21910251.4
(22) Date of filing: 06.12.2021
(51) Int. Cl.: C12M 3/00, C12N 5/077

(54) **CULTURE MEMBER AND USE THEREOF**

(30) Priority: 23.12.2020 JP 2020213523
(71) Applicant: Mitsui Chemicals, Inc., Tokyo 104-0028 (JP)
(72) Inventor: ESASHIKA, Katsuhiro, Sodegaura-shi, Chiba 299-0265 (JP); MIYASAKO, Hiroshi, Mobara-shi, Chiba 297-0017 (JP); KOZEKI, Takashi, Sodegaura-shi, Chiba 299-0265 (JP); AMANO, Koh, Mobara-shi, Chiba 297-0017 (JP); SAIJO, Yoshinobu, Mobara-shi, Chiba 297-0017 (JP); YANAI, Hisaaki, Mobara-shi, Chiba 297-0017 (JP)
(74) Representative: J A Kemp LLP
(86) International application number: PCT/JP2021/044665
(87) International publication number: WO 2022/138101

(57) **Abstract**

Provided is a culture material and a culture tool, which allow to culture, cells, etc. to form a spheroid.

A culture material for culturing cells, a tissue, or an organ on a culture surface thereof, the culture material containing a 4-methyl-1-pentene polymer (X), having a water contact angle of the culture surface of more than 100° and 160° or less, and having an oxygen permeability at a temperature of 23°C and humidity of 0% of 4500 to 90000 cm³/ (m²×24h×atm).

## Description

### TECHNICAL FIELD

The present invention relates to a culture material and a use thereof.

### BACKGROUND ART

Organs and tissues of living organisms express their functions by having their constituent cells form a three-dimensional network. Therefore, for the purpose of fully exhibiting the inherent functions of the cells, spheroids obtained by culturing cells in three dimensions, as opposed to the conventional two-dimensional cell culture, have been produced, and are gaining attention. Spheroids have been reported to provide results closer to living organisms than two-dimensionally cultured cells in the evaluation of cell function and drug screening, for example, and are expected to be an important tool for bridging the gap between *in vitro* and *in vivo* testing in drug development. In addition, stem cells, including iPS cells, are expected to be an important source of cells for regenerative medicine and cell therapy, which requires the preparation of large quantities of high-quality cells. Culturing stem cells in three dimensions to form spheroids allows to culture at high density while maintaining the original pluripotency (undifferentiated state) of the cells, and therefore the usefulness of spheroids is also gaining attention in regenerative medicine. Accordingly, there is a growing demand for novel cell culture techniques to obtain cell spheroids of uniform shape and size in large quantities, and in a stable and simple manner.

For the above reasons, various methods for forming spheroids have been developed. Examples thereof include a method using a culture vessel whose surface is processed or treated to inhibit cell adhesion (Patent Literature 1), a method of culturing in a free-floating state in a vessel in which a resin layer with low cell adhesiveness is formed (Patent Literature 2), and a method of forming spheroids adhered to the surface of a culture vessel by setting the amount of proteoglycan adsorbed on the culture vessel above a specific value (Patent Literature 3). In particular, when culturing non-adherent cells to form spheroids, a treatment to prevent cells from adhering to the surface of the culture vessel (e.g., making the surface of the culture vessel superhydrophilic, hydrophobic, or structured to prevent adhesion) is usually performed.

### CITATION LIST

### PATENT LITERATURE

Patent Literature 1: WO2020/013345
Patent Literature 2: JP2008-061609A
Patent Literature 3: JP2017-77241A

### SUMMARY OF INVENTION

### TECHNICAL PROBLEM

The present invention was made in consideration of the above circumstances, and addresses the problem of providing a culture material and a culture tool, which allow to culture cells, etc. to form a spheroid. The present invention also addresses the problem of providing a culture material and a culture tool that have excellent shape stability, are capable of achieving an oxygen environment suitable for the culture of cells, tissues, or organs, do not emit autofluorescence and impair the observability of the cells, and is less subject to drug sorption. Furthermore, the present invention addresses the problem of providing a culture material and a culture tool which allow to culture differentiated cells derived from stem cells in particular, with a high degree of differentiated cell purity.

### SOLUTION TO PROBLEM

The present inventors have conducted diligent research in order to solve the problems described above. As a result, they have found that the problems described above can be solved by, for example, a culture material having the following configuration, thereby completing the present invention. The present invention is, for example, the following [1] to [12].
[1] A culture material for culturing cells, a tissue, or an organ on a culture surface thereof, the culture material containing a 4-methyl-1-pentene polymer (X), having a water contact angle of the culture surface of more than 100° and 160° or less, and having an oxygen permeability at a temperature of 23°C and humidity of 0% of 4500 to 90000 cm³/(m²×24h×atm).
[2] The culture material according to [1], wherein the 4-methyl-1-pentene polymer (X) is a copolymer (x1) of 4-methyl-1-pentene and at least one selected from ethylene and α-olefins having 3 to 20 carbon atoms (excluding 4-methyl-1-pentene).
[3] The culture material according to [1] or [2], wherein the culture surface is not processed to form an uneven structure.
[4] The culture material according to any of [1] to [3], which is for spheroid formation.
[5] The culture material according to any of [1] to [4], wherein the cells, tissue, or organ include differentiated cells derived from iPS cells.
[6] The culture material according to any of [1] to [5], wherein the cells, tissue, or organ include cardiomyocytes.
[7] The culture material according to any of [1] to [6], wherein the shape of the culture material is in the form of a film or a sheet.
[8] A culture tool, wherein at least the culture surface is formed of the culture material according to any of [1] to [7] .
[9] A method for culturing cells, a tissue, or an organ, the method including:
   a step (A) of contacting the cells, tissue, or organ with the culture surface of the culture material according to any of [1] to [7] or the culture tool according to [8]; and
   a step (B) of culturing the cells, tissue, or organ in contact with the culture surface to form a spheroid.
[10] The method for culturing cells, a tissue, or an organ according to [9], further including a step (C) of inducing differentiation of the cells, etc., wherein the step (C) is a step of inducing differentiation of iPS cells into cardiomyocytes by the protein-free cardiac differentiation (PFCD) method.
[11] The method for culturing cells, a tissue or an organ according to [9] or [10] that increases cardiac purity.
[12] A spheroid formed by the culture method according to any of [9] to [11].

### ADVANTAGEOUS EFFECTS OF INVENTION

According to the present invention, it is possible to provide a culture material and a culture tool, which allow to culture cells, etc. to form a spheroid. According to the present invention, it is also possible to provide a culture material and a culture tool that have excellent shape stability, are capable of achieving an oxygen environment suitable for the culture of cells, tissues, or organs, do not emit autofluorescence and impair the observability of the cells, and is less subject to drug sorption. Furthermore, according to the present invention, it is possible to provide a culture material and a culture tool which allow to culture differentiated cells derived from stem cells in particular, with a high degree of differentiated cell purity. In addition, the culture material and culture tool of the present invention facilitate the differentiation of iPS cells into cardiomyocytes.

### BRIEF DESCRIPTION OF DRAWINGS

[Figure 1] Figure 1 is a graph showing an experimental schedule for inducing differentiation of iPS cells into cardiomyocytes.
[Figure 2] Figure 2 is a photograph of the observation of cell morphology on day 12 of induction of cardiac differentiation.
[Figure 3] Figure 3 is a photograph of the observation of cell morphology on day 19 of induction of cardiac differentiation.
[Figure 4] Figure 4 is a photograph of the observation of human osteosarcoma-derived cancer cells (HOF-143B) from Example 3 forming spheroids.

### DESCRIPTION OF EMBODIMENTS

Unless otherwise specified, the statement "A to B" relating to a numerical range indicates that it is A or more and B or less. For example, the statement "1 to 5%" means 1% or more and 5% or less.

### [Culture Material]

The culture material according to the present invention is a culture material for culturing cells, a tissue, or an organ (hereinafter also referred to as cells, etc.) on a culture surface thereof, the culture material being characterized in that it contains a 4-methyl-1-pentene polymer (X), has a water contact angle of the culture surface of more than 100° and 160° or less, and has an oxygen permeability at a temperature of 23°C and humidity of 0% of 4500 to 90000 cm³/(m²×24h×atm).

Here, a culture material means a material that constitutes at least a part of a culture tool used to culture cells, etc. When the culture material is part of the culture tool, at least the culture surface on which the cells, etc. are cultured is composed of the culture material of the present invention. Here, the culture surface means the surface on which the culture medium is formed, the surface on which the cells, etc. are seeded, or the surface on which the culture medium is formed and the cells, etc. are seeded when culturing cells, etc. That is, the culture surface is a concept including the surface on which the culture medium is to be formed and the surface on which the cells, etc. are to be seeded.

In the present specification, culture is used in a broad sense that includes not only the growth and maintenance of cells, etc., but also processes such as the seeding, passaging, induction of differentiation, and induction of self-organization of cells, etc.

The form of the culture material of the present invention is not particularly limited, and may be, for example, of a film or a sheet. When the culture material is in the form of a film or a sheet, it can be suitably used as a culture tool, with at least one side of the culture material in the form of a film or a sheet used as the culture surface.

The culture material of the present invention means one in which the culture surface is not coated with a natural polymer material, synthetic polymer material, or inorganic material to serve as an anchorage for the cells, etc.

The thickness of the culture material of the present invention is not particularly limited. The thickness of the culture material of the present invention is not particularly limited, but 20 to 500 um is preferred, 25 to 400 um is more preferred, and 50 to 200 um is particularly preferred. A thickness of the culture material within the above range allows to obtain the appropriate oxygen concentration in the culture medium necessary for cell growth, and to more easily produce a suitable culture tool without the culture material, particularly the bottom of the culture vessel being warped.

The thickness of the culture material of the present invention when the culture material is placed on the bottom of a vessel to produce a culture tool such as a dish (also referred to as a petri dish), flask, insert, or plate is not particularly limited, but is preferably 20 um to 400 µm, more preferably 20 um to 300 µm, and even more preferably 20 um to 200 um thick. The thickness of the culture material is appropriately selected according to the form of the culture tool, but adjusting it to the above range allows to more easily obtain the appropriate oxygen concentration in the culture medium necessary for cell growth, and to more easily produce a suitable culture tool having sufficient strength.

The surface of the culture material of the present invention may be processed as long as the effects of the present invention are not impaired. Examples of processing of the surface include processing to form uneven structures, and surface modification treatments such as hydrophilic treatment and hydrophobic treatment.

Even without processing its surface, the culture material of the present invention allows the formation of spheroids because cells do not easily adhere to it. Therefore, the surface of the culture material of the present invention is preferably not processed, and more preferably not processed to form an uneven structure.

The method used for the surface modification treatment is not particularly limited, but examples thereof include hydrophilic treatments such as corona treatment, plasma treatment, ozone treatment, and ultraviolet treatment; hydrophobic treatments such as esterification, silylation, and fluorination; surface graft polymerization, chemical vapor deposition, etching, or the addition of specific functional groups such as hydroxyl groups, amino groups, sulfone groups, thiol groups, and carboxyl groups; treatments with specific functional groups such as silane coupling, titanium coupling, and zirconium coupling; and physical treatments such as surface roughening by oxidizing agents, for example, rubbing and sandblasting. These surface modification treatments may be performed singly, or two or more may be performed in combination. When a surface modification treatment is performed, it is preferably performed at least on the culture surface.

The method for producing the culture material of the present invention is not particularly limited, nor is the equipment used for production. For example, a film or sheet containing a 4-methyl-1-pentene polymer (X) can be formed, and if necessary, the film or sheet can be molded to produce the culture material as a molded article in the desired shape. The film, sheet, or other molded article serving as the culture material can also be obtained by direct molding by a method such as extrusion molding, solution casting, injection molding, and blow molding.

Specifically, as a method for forming the film or sheet, for example, the usual inflation method or T-die extrusion method are employed. Production is usually performed with heating. When the T-die extrusion method is employed, the extrusion temperature is preferably 100°C to 400°C, and particularly preferably 200°C to 300°C. In addition, the roll temperature is preferably 45°C to 75°C, and particularly preferably 55°C to 65°C.

The film or sheet may also be produced by a solution casting method, in which the 4-methyl-1-pentene polymer (X) is dissolved in a solvent, poured onto a resin or metal, and slowly dried while leveling to form a film (sheet). The solvent used is not particularly limited, and hydrocarbon solvents such as cyclohexane, hexane, decane, and toluene may be used. Two or more solvents may also be mixed in consideration of the solubility and drying efficiency of the 4-methyl-1-pentene polymer (X). The polymer solution can be applied by a method such as table coating, spin coating, dip coating, die coating, spray coating, bar coating, roll coating, and curtain flow coating, then dried and peeled off to be processed into a film or sheet.

The culture material of the present invention is preferably a culture material for forming spheroids, and more preferably a cell culture material for forming spheroids.

### [4-Methyl-1-pentene Polymer]

In the present invention, the 4-methyl-1-pentene homopolymer and the copolymers of 4-methyl-1-pentene and other monomers are collectively referred to as the "4-methyl-1-pentene polymer (X)".

The copolymer of 4-methyl-1-pentene and another monomer, which is an example of the 4-methyl-1-pentene polymer, may be any of a random copolymer, an alternating copolymer, a block copolymer, or a graft copolymer. As a copolymer of 4-methyl-1-pentene and another monomer, a copolymer of 4-methyl-1-pentene and at least one olefin selected from ethylene and α-olefins having 3 to 20 carbon atoms (excluding 4-methyl-1-pentene) is preferred as it has high strength, is less likely to break or crack even when used as a material, and has little warping.

The 4-methyl-1-pentene polymer is preferably a 4-methyl-1-pentene homopolymer, and at least one polymer selected from copolymers of 4-methyl-1-pentene and at least one olefin selected from ethylene and α-olefins having 3 to 20 carbon atoms (excluding 4-methyl-1-pentene), and more preferably a copolymer of 4-methyl-1-pentene and at least one olefin selected from ethylene and α-olefins having 3 to 20 carbon atoms (excluding 4-methyl-1-pentene).

Examples of the olefin include ethylene, propylene, 1-butene, 1-hexene, 1-heptene, 1-octene, 1-decene, 1-tetradecene, 1-hexadecene, 1-heptadecene, 1-octadecene, and 1-eicosene. The olefin can be appropriately selected according to the physical properties necessary for the culture material. For example, from the viewpoint of appropriate oxygen permeability and excellent stiffness, the olefin is preferably an α-olefin having 8 to 18 carbon atoms, and more preferably at least one selected from 1-octene, 1-decene, 1-dodecene, 1-tetradecene, 1-hexadecene, 1-heptadecene and 1-octadecene. When the number of carbons of the olefin is within the above range, the processability of the polymer tends to be better and defects in the appearance of the culture material due to cracks and cracked edges are less likely to occur. In addition, the incidence of defective culture materials is reduced.

One or more of the above olefins can be used. From the viewpoint of material strength, the number of carbon atoms is preferably 2 or more, and more preferably 10 or more. When combining two or more different α-olefins, it is particularly preferred to combine at least one selected from 1-tetradecene and 1-hexadecene, with at least one selected from 1-heptadecene and 1-octadecene.

The content of structural units derived from 4-methyl-1-pentene in the 4-methyl-1-pentene polymer is preferably 60 to 100 mol%, and more preferably 80 to 98 mol%.

In addition, when the 4-methyl-1-pentene polymer is a copolymer of 4-methyl-1-pentene and at least one olefin selected from ethylene and α-olefins having 3 to 20 carbon atoms (excluding 4-methyl-1-pentene), the content of structural units derived from at least one olefin selected from ethylene and α-olefins having 3 to 20 carbon atoms (excluding 4-methyl-1-pentene) in the copolymer is preferably 0 to 40 mol%, and more preferably 2 to 20 mol%. The content of these structural units is based on the total amount of repeating structural units in the 4-methyl-1-pentene polymer as 100 mol%. When the content of structural units is within the above range, a homogeneous culture surface with excellent processability is obtained, and there is less warping as the film has a good balance between toughness and strength.

The 4-methyl-1-pentene polymer may have structural units other than the structural units derived from 4-methyl-1-pentene and the structural units derived from the ethylene and α-olefins having 3 to 20 carbon atoms (hereinafter also referred to as "other structural units"), as long as the effects of the present invention are not impaired. The content of other structural units is, for example, 0 to 10.0 mol%. When the 4-methyl-1-pentene polymer has other structural units, the other structural units may be of one type or two or more types.

Examples of monomers from which the other structural units are derived include cyclic olefins, aromatic vinyl compounds, conjugated dienes, non-conjugated polyenes, functional vinyl compounds, hydroxyl-containing olefins, and halogenated olefins. As the cyclic olefins, aromatic vinyl compounds, conjugated dienes, non-conjugated polyenes, functional vinyl compounds, hydroxyl-containing olefins, and halogenated olefins, for example, the compounds described in paragraphs [0035] to [0041] of JP2013-169685A can be used.

The 4-methyl-1-pentene polymer may be used singly, or two or more may be used in combination.

Commercial products can also be used as the 4-methyl-1-pentene polymer. Specific examples include TPX MX001, MX002, MX004, MX0020, MX021, MX321, RT18, RT31 or DX845 (all trademarks) manufactured by Mitsui Chemicals, Inc. Other manufacturers can also be preferably used as long as it is a 4-methyl-1-pentene polymer meeting the above requirements. These commercial products may be used singly, or two or more may be used in combination.

The 4-methyl-1-pentene polymer usually has a melting point of 200°C to 240°C and high heat resistance. Since it does not undergo hydrolysis and has excellent water resistance, boiling water resistance, and steam resistance, the culture material, for example, the culture tool containing the 4-methyl-1-pentene polymer can be sterilized with high pressure steam. Since the 4-methyl-1-pentene polymer also has high visible light transmittance (usually 90% or more) and is characterized by not emitting autofluorescence, the culture tool containing the 4-methyl-1-pentene polymer allows easy observation of the cultured cells. Furthermore, since the 4-methyl-1-pentene polymer exhibits excellent chemical resistance to most chemicals and is less subject to drug sorption, it is also suitably used in applications of drug discovery screening and diagnosis. The 4-methyl-1-pentene polymer is heat sealable and is not only easily thermally fused to itself, but also easily thermally bonded to other materials as well. In addition, since it can be thermoformed, it is easily molded into culture tools of any shape, and for example, it is also easily molded using the imprinting or insert method.

The weight average molecular weight (Mw) of the 4-methyl-1-pentene polymer, as measured by gel permeation chromatography (GPC) using standard polystyrene as the reference material, is preferably 10000 to 2000000, more preferably 20000 to 1000000, and even more preferably 30000 to 500000. Here, the sample concentration for GPC measurement can be, for example, 1.0 to 5.0 mg/ml. In addition, the molecular weight distribution (Mw/Mn) of the 4-methyl-1-pentene polymer is preferably 1.0 to 30, more preferably 1.1 to 25, and even more preferably 1.1 to 20. O-dichlorobenzene is preferably used as the solvent used in GPC. Examples of the measurement conditions include the conditions shown in the Examples described below, but are not limited to these measurement conditions.

In the molding method for the 4-methyl-1-pentene polymer described below, for a film produced by melt molding, setting the weight average molecular weight (Mw) to the above upper limit or less allows to more easily suppress the occurrence of defects such as gel and to more easily form a film with a uniform surface. In addition, when produced by the solution casting method, it allows to improve the solubility in the solvent, to more easily suppress defects of the film such as gel and to form films with a uniform surface.

By setting the weight average molecular weight (Mw) to the above lower limit or more, the culture material tends to have sufficient strength. Furthermore, by setting the molecular weight distribution within the above range, the stickiness of the surface of the produced culture material is more easily suppressed, and the toughness of the culture material tends to be sufficient, which allows to more easily suppress the occurrence of bending during molding and cracking during cutting, for example.

As for the weight average molecular weight (Mw) and molecular weight distribution (Mw/Mn) of the 4-methyl-1-pentene polymer, when two or more 4-methyl-1-pentene polymers are used, the Mw and Mw/Mn of each may be within the above ranges.

Having the above excellent properties, the 4-methyl-1-pentene polymer is exceptional as a material for culture material, since the culture tool in which at least the culture surface is formed of the culture material of the present invention does not cause any adverse effect on the culture, has good stability, light permeability, and molding processability, and can be sterilized.

### [Method for Producing 4-Methyl-1-pentene Polymer]

The method for producing the 4-methyl-1-pentene polymer may be any method as long as it can polymerize 4-methyl-1-pentene, olefins, and other monomers. A chain transfer agent, for example, hydrogen, may coexist to control the molecular weight and molecular weight distribution. The equipment used for production is also not limited. The polymerization method may be a known method, and may be a gas-phase method, slurry method, solution method, or bulk method. A slurry or solution method is preferred. The polymerization method may be a single-stage polymerization method or a multistage, for example, two-stage polymerization method, and may be a method in which a plurality of polymers with different molecular weights are blended in the polymerization system. Whether in single-stage or multistage polymerization, if hydrogen is used as a chain transfer agent, it may be charged in batch or in divided amounts, for example, at the beginning, middle, and end of polymerization. Polymerization may be performed at ordinary temperatures, or with heating if necessary, but from the viewpoint of polymerization efficiency, it is preferably performed at 20°C to 80°C, and particularly preferably at 40°C to 60°C. The catalyst used for production is also not limited, but from the viewpoint of polymerization efficiency, for example, the solid titanium catalyst component (I) described in WO2006/054613, or the catalyst for olefin polymerization (metallocene catalyst) containing the transition metal compound (A) described in WO2014/050817 is preferably used.

When the culture material is formed from a composition containing the 4-methyl-1-pentene polymer (X), the 4-methyl-1-pentene polymer (X) is preferably 90 mass% or more and less than 100 mass%, more preferably 95 mass% or more and less than 100 mass%, and particularly preferably 99 mass% or more and less than 100 mass% per 100 mass% of the culture material. Containing a large amount of components other than the 4-methyl-1-pentene polymer (X) leads not only to a decrease in oxygen permeability, but also to a decrease in transparency and strength.

The material forming the culture material of the present invention may contain components other than the 4-methyl-1-pentene polymer (X). Examples of components other than the 4-methyl-1-pentene polymer (X) include additives such as heat-resistant stabilizers, light-resistant stabilizers, processing aids, plasticizers, antioxidants, lubricants, defoaming agents, antiblocking agents, colorants, modifiers, antibacterial agents, antifungal agents, and antifogging agents.

### [Water Contact Angle]

The culture material of the present invention has a water contact angle of the culture surface thereof of more than 100° and 160° or less. The water contact angle is preferably more than 100° and 150° or less, more preferably more than 100° and 130° or less, and even more preferably more than 105° and 130° or less. If the water contact angle of the culture surface of the culture material is 100° or less, the cells, etc. will adhere to the culture material, making it difficult to form spheroids. If the water contact angle of the culture surface of the culture material is more than 160°, the contact between the culture surface and the culture medium will not be sufficient, which will decrease the efficiency of oxygen supply into the culture medium.

The method for measuring the water contact angle is not particularly limited, and a known method can be used, but it is preferably the sessile drop method. The water contact angle can be measured, for example, by a method of adding dropwise a drop of water with a volume of 4 µL or less, the shape of which can be considered as spherical, on the surface of the culture material or a measurement sample produced using the same material as the culture material, under constant temperature and humidity conditions of 25 ± 5°C and 50 ± 10%, in accordance with the Japanese Industrial Standard JIS-R3257 (Testing method of wettability of the surface of glass substrate), and then using the sessile drop method, measuring the angle of the contact interface between the measurement sample and the drop of water within one minute immediately after the drop of water makes contact with the surface of the measurement sample.

### [Oxygen Permeability]

The oxygen permeability of the culture material of the present invention at a temperature of 23°C and humidity of 0% is 4500 to 90000 cm³/(m²×24h×atm), preferably 4500 to 67500 cm³/(m²×24h×atm), more preferably 4500 to 47000 cm³/(m²×24h×atm), and even more preferably 4500 to 45000 cm³/(m²×24h×atm).

If the oxygen permeability of the culture material is too low, the oxygen concentration in the culture medium will be low and the cells will not grow sufficiently. On the other hand, if the oxygen permeability is too high, the oxygen concentration in the medium will be too high, which will reduce cell function due to oxygen stress. When the oxygen permeability is within the above upper and lower limits, the cells can maintain good morphology and grow efficiently according to the culture period.

The oxygen permeability coefficient [cm³×mm/(m²×24h×atm)] at a temperature of 23°C and humidity of 0% of the culture material or a measurement sample produced using the same material as the culture material is measured by a differential pressure gas permeability measurement method, and the value obtained by dividing the oxygen permeability coefficient by the thickness (µm) of the culture material is used as the oxygen permeability [cm³/(m²×24h×atm)]. The equipment used for the measurement is not particularly limited as long as it uses a differential pressure gas permeability measurement method, and examples thereof include the differential pressure gas permeability measuring device MT-C3 manufactured by Toyo Seiki Seisaku-sho, LTD. It is preferable that the measurement sample be produced by cutting a 90×90 mm test piece from a 50 um thick film, for example, and that the diameter of the measurement section be 70 mm (permeation area: 38.46 cm²). Because of the large oxygen permeability, it is more preferable to cover the sample with an aluminum mask beforehand to have an actual permeation area of 5.0 cm². The culture material, or measurement sample produced using the same material as the culture material, that is used to measure oxygen permeability may or may not have undergone microfabrication or a surface modification treatment, but preferably has not undergone any treatment.

### [Cells, tissue, or organ]

In the present specification, the cells, tissue, or organ are also simply referred to as "cells, etc.". The origin of the cells, etc. is not particularly limited and may be of any organism, such as an animal, a plant, an insect, a fungus, a protist, or a bacterium, but an animal or a plant is preferred, an animal is even more preferred, and a mammal is particularly preferred. Since the culture material of the present invention allows to culture while avoiding the adhesion of cells, etc. and also has excellent oxygen permeability, the cells, etc. are preferably non-adherent.

The cells in the present invention are not particularly limited, and examples thereof include plant cells, animal cells, and insect cells, but they are preferably animal cells, and more preferably mammalian cells. As the mammalian cells, cells derived from humans, monkeys, mice, rats, pigs, dogs, sheep, cats, and goats are preferred, and cells derived from humans are more preferred. The cells may be cells cultured in two dimensions, or cells cultured in three dimensions, and include spheroids obtained by culturing cells. As the cells in the present invention, frozen or refrozen cells may be used. In addition, as the cells in the present invention, passaged cells may be used, and the number of passages is not particularly limited.

As the animal cells, normal cells, cancer cells, and fusion cells such as hybridomas can be used, and they may be cells that have undergone artificial treatment such as gene transfer. The animal cells may be either primary cultured cells or a subcultured cell line. The animal cells may be free-floating cells or adherent cells. Examples of animal cells include undifferentiated pluripotent stem cells, differentiated cells derived from pluripotent stem cells (including pluripotent stem cells for which induction of differentiation has been initiated and which are already in the process of differentiation), undifferentiated somatic stem cells, differentiated cells derived from somatic stem cells (including somatic stem cells for which induction of differentiation has been initiated and which are already in the process of differentiation), and differentiated cells derived from animal tissue.

In the present invention, differentiated cells are not limited to cells matured to the final stage of differentiation (terminally differentiated mature cells). The differentiated cells may be cells that differentiate and mature from the ectoderm, cells that differentiate and mature from the mesoderm, or cells that differentiate and mature from the endoderm.

Pluripotent stem cells mean cells that have pluripotency, which is the ability to differentiate into all the cells that make up a living organism, and self-renewal capacity, which is the ability to maintain their pluripotency through cell division. Pluripotent stem cells include embryonic stem cells (ES cells), embryonic germ cells (EG cells), induced pluripotent stem cells (iPS cells), muse cells (multi-lineage differentiating stress enduring cells), embryonic carcinoma cells (EC cells), trophoblast stem cells (TS cells), and epiblast stem cells (EpiS cells). Pluripotent stem cells are preferably ES cells or iPS cells, and more preferably iPS cells.

ES cells can be established by removing the inner cell mass from a blastocyst of a mammalian fertilized egg and culturing the inner cell mass on a fibroblast feeder. Maintenance of the cells by subculture can be performed using a culture solution supplemented with substances such as leukemia inhibitory factor (LIF) and basic fibroblast growth factor (bFGF). The methods for establishing and maintaining human and monkey ES cells are described, for example, in USP5,843,780; Thomson JA, et al. (1995), Proc Natl. Acad. Sci. U S A. 92:7844-7848; Thomson JA, et al. (1998), Science. 282:1145-1147; H. Suemori et al. (2006), Biochem. Biophys. Res. Commun., 345:926-932; M. Ueno et al. (2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99:1580-1585; and Klimanskaya I, et al. (2006), Nature. 444:481-485.

Human ES cell lines, for example, WA01 (H1) and WA09 (H9) are available from WiCell Research Institute, and KhES-1, KhES-2 and KhES-3 are available from the Institute for Frontier Life and Medical Sciences, Kyoto University (Kyoto, Japan).

iPS cells can be produced by introducing specific reprogramming factors into somatic cells in the form of DNA or proteins (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al, Nat. Biotechnol. 26:101-106 (2008); and WO2007/069666).

The somatic cells mean any animal cells (preferably mammalian cells, including human) except germ line cells or differentiated totipotent cells such as ova, oocytes, and ES cells, and include fetal somatic cells, neonatal somatic cells, and mature healthy or diseased somatic cells, as well as primary cultured cells, passaged cells, and established cells. Examples of the somatic cells include tissue stem cells (somatic stem cells) such as neural stem cells, hematopoietic stem cells, mesenchymal stem cells, and dental pulp stem cells; tissue progenitor cells; and differentiated cells such as lymphocytes, epithelial cells, endothelial cells, muscle cells, fibroblasts, hair cells, hepatocytes, gastric mucosal cells, enterocytes, splenocytes, pancreatic cells, brain cells, lung cells, renal cells and adipocytes.

The reprogramming factor may consist of a gene specifically expressed in ES cells, a gene product or noncoding RNA thereof, or a gene that plays an important role in maintaining ES cells undifferentiated, a gene product or noncoding RNA thereof, or a low molecular compound. Examples of the genes included in the reprogramming factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tell, beta-catenin, Lin28b, Sall1, Sall4, Esrrb, Nr5a2, Tbx3, and Glis1, and these reprogramming factors may be used singly, or two or more may be used in combination.

Somatic stem cells mean cells that have limited differentiation potential, which is the ability to differentiate into specific cells, and self-renewal capacity, which is the ability to maintain their limited differentiation potential through cell division. Somatic stem cells include mesenchymal stem cells, hematopoietic stem cells, and neural stem cells.

Examples of differentiated cells derived from animal tissues include adipocytes, hepatocytes, renal cells, pancreatic cells, mammary gland cells, endothelial cells, epithelial cells, smooth muscle cells, myoblasts, cardiomyocytes, nerve cells, glial cells, dendritic cells, chondrocytes, osteoblasts, osteoclasts, osteocytes, fibroblasts, the various blood cells, retinal cells, corneal cells, gonadal cells, and the various glandular cells, including the various progenitor cells.

The cells may be used singly, or two or more may be used in combination.

The animal cells are preferably pluripotent stem cells and differentiated cells derived from pluripotent stem cells, more preferably iPS cells and differentiated cells derived from iPS cells, even more preferably cardiomyocytes derived from iPS cells, and particularly preferably human cardiomyocytes derived from iPS cells, as they are suitable for spheroid formation. Alternatively, the animal cells are preferably somatic stem cells, and more preferably mesenchymal stem cells, as they are suitable for spheroid formation. Alternatively, the animal cells are preferably cancer cells, as they are suitable for spheroid formation. Alternatively, the animal cells are preferably cells that are generally required to be cultured in three dimensions, more preferably cells that form spheroids, such as hepatocytes, nerve cells, cardiomyocytes, pancreatic β cells, vascular endothelial cells, adipocytes, adipose-derived stem cells, chondrocytes, mesenchymal stem cells, epithelial hair follicle stem cells, follicle dermal papilla cells, skin fibroblasts, skin keratinocytes, and osteoblasts, as the conditions of the cells can be more approximated to those *in vivo* which have a three-dimensional structure.

If the animal cells are differentiated cells derived from pluripotent stem cells, the cells can be produced from pluripotent stem cells by a known method for inducing differentiation. Alternatively, differentiated cells derived from pluripotent stem cells that are commercially available may be used. If the animal cells are somatic stem cells, those harvested from animals or commercially available ones may be used.

The differentiated cells derived from pluripotent stem cells are preferably cardiomyocytes derived from iPS cells.

The cardiomyocytes derived from iPS cells can be produced, for example, by the known protein-free cardiac differentiation (PFCD) method (see WO2015/182765). Since the protein-free cardiac differentiation (PFCD) method can achieve a high efficiency of cardiac differentiation, the cardiomyocytes derived from iPS cells produced by this method can achieve high cardiomyocyte purity.

In the present invention, a spheroid refers to a mass of cells gathered together and can be paraphrased, for example, as a cell aggregate or cell mass. The spheroid may be a spheroid containing a single cell type, such as cardiomyocytes, or a spheroid containing two or more different cell types, such as various fibroblasts or vascular endothelial cells, for example, and cardiomyocytes. Examples of cells that can be used include the above various types of cells. The spheroid formed using the culture material or culture tool of the present invention is preferably a spheroid containing pluripotent stem cells and a spheroid containing differentiated cells derived from pluripotent stem cells, more preferably a spheroid containing iPS cells or a spheroid containing differentiated cells derived from iPS cells, and even more preferably a spheroid containing cardiomyocytes derived from iPS cells.

The spheroid preferably has a differentiated cell purity (number of differentiated cells/number of cells constituting the spheroid × 100) of preferably 10% or more, more preferably 13% or more, even more preferably 15% or more, still more preferably 20% or more, and particularly preferably 30% or more. When iPS cells are induced to differentiate and form spheroids with cardiomyocytes as the target cells, the spheroids contain cardiomyocytes derived from iPS cells at a cardiomyocyte purity of preferably 10% or more, more preferably 20% or more, and even more preferably 30% or more. The number of differentiated cells can be determined by a known method, for example, by flow cytometric analysis using antibodies against, for example, cardiac troponin T (cTnT), troponin, MYL2 (myosin regulatory light chain 2) and MYL7 (myosin regulatory light chain 7), which are myocardial markers. The number of cells constituting the spheroid can be determined by a known method, for example, by treating the spheroid with trypsin to dissociate it into single cells and counting the number of single cells.

When pluripotent stem cells are differentiated to obtain differentiated cells, the spheroid obtained in the end will also contain some amount of undifferentiated cells or cells that have undergone differentiation other than targeted differentiation, in addition to the differentiated target cells. Therefore, when the differentiated cell purity is within the above range, the reliability and reproducibility of the data when spheroids of differentiated cell are used for various tests are more likely to be high.

The size of the spheroid is not particularly limited. Although the size of the spheroid depends, for example, on the type of cells forming the spheroid, the number of cells, the culture medium, and the number of days of culture, its average diameter is, for example, preferably 10 to 10000 µm, more preferably 10 to 8000 µm, and even more preferably 10 to 5000 um. The diameter of the spheroid can be determined, for example, by a method of observing them under a microscope and measuring the diameter on a photograph taken, or by a method of using a particle size analyzer.

The number of cells constituting a spheroid is not particularly limited. While depending, for example, on the type of cells forming the spheroid, the culture medium, and the number of days of culture, each spheroid may contain, for example, 1 × 10¹ cells or more, 1 × 10² cells or more, 1 × 10³ cells or more, 1 × 10⁴ cells or more, 1 × 10⁵ cells or more, 1 × 10⁶ cells or more, 1 × 10⁷ cells or more, 1 × 10⁸ cells or more, or 1 × 10⁹ cells or more. The upper limit thereof is not particularly set. The number of cells constituting a spheroid can be calculated, for example, from the fluorescence intensity after cell staining with a fluorescent reagent, and a calibration curve of the cell number and fluorescence intensity.

The culture medium used for cell culture may be appropriately selected according to the cells. The type of culture medium is not particularly limited, but for example, any cell culture basal medium, differentiation medium, or medium dedicated to primary culture can be used. Specific examples include Essential 8, Eagle's Minimum Essential Medium (EMEM), Dulbecco's Modified Eagle's Medium (DMEM), α-MEM, Glasgow's MEM (GMEM), IMDM, RPMI 1640, Ham's F-12, MCDB medium, William's medium E, Hepatocyte thaw medium, and mixed media thereof, but are not limited thereto, and any medium can be used as long as it contains the components necessary for cell growth and differentiation. Furthermore, a culture medium supplemented with, for example, serum, various growth factors, differentiation inducing factors, antibiotics, hormones, amino acids, sugars, or salts may be used. The culture temperature is also not particularly limited, but the culture is usually performed at about 25 to 40°C.

The amount of culture medium is not particularly limited, but the height of the culture medium is preferably 3 to 30 mm, more preferably 3 to 25 mm, and even more preferably 4 to 20 mm.

A tissue in the present invention means a collection of similar cells that perform similar functions, and is a different concept from spheroids. The tissue is not particularly limited, and examples thereof include epithelial tissue, connective tissue, muscle tissue, and nerve tissue. The tissue is preferably tissue that contains cells forming spheroids, and examples of such tissue include nerve tissue containing nerve cells, cardiac muscle tissue containing cardiomyocytes, adipose tissue containing adipocytes or adipose-derived stem cells, cartilage tissue containing chondrocytes, and bone tissue containing osteoblasts. Of these, the tissue is preferably nerve tissue or cardiac muscle tissue, and even more preferably cardiac muscle tissue because of their high oxygen demand. The tissue is also preferably tissue containing somatic stem cells, as it is suitable for spheroid formation.

An organ in the present invention means a collection of the above tissues that work together for a purpose. The organ is not particularly limited, and examples thereof include lung, heart, liver, kidney, spleen, pancreas, gallbladder, esophagus, stomach, skin, and brain. The organ is preferably an organ that contains cells forming spheroids, and examples of such organ include liver containing hepatocytes, pancreas containing pancreatic β cells, blood vessels containing vascular endothelial cells, bone marrow containing mesenchymal stem cells, hair follicles containing epithelial hair follicle stem cells or follicle dermal papilla cells, kidney, and skin containing skin fibroblasts or skin keratinocytes. Of these, the organ is preferably the skin, kidney, liver, pancreas, heart, or hair follicle, and even more preferably the heart because of their high oxygen demand. The organ is also preferably an organ containing somatic stem cells, as it is suitable for spheroid formation.

### [Culture Tool]

In the present invention, a culture tool means all instruments used for culturing cells, etc. The culture tool is composed, at least in part, of the culture material. The culture tool may be composed entirely of the culture material, or only a part thereof may be composed of the culture material. When only a part of the culture tool is composed of the culture material, at least the culture surface on which cells, etc. are cultured is composed of the culture material of the present invention.

The culture tool is usually used in an apparatus such as an incubator, a mass culture apparatus, or a perfusion culture apparatus.

As the culture tool, various types of known culture tools can be used, and their shapes and sizes are not particularly limited. Examples of the culture tool include culture vessels such as dishes, flasks, plates, bottles, bags, and tubes, as well as inserts, cups, liners, and slides, and it is preferably a culture vessel.

The culture tool may be a culture tool having at least one well or a culture vessel having at least one well. A culture vessel having at least one well is, for example, a plate having at least one well, more specifically, a plate having, for example, 6 wells, 12 wells, 24 wells, 48 wells, 96 wells, 384 wells, or 1536 wells. Generally, a culture tool having a well-like hollow shape on its bottom surface requires a thicker bottom surface to stabilize the complex shape of the bottom surface, which makes it difficult to sufficiently supply oxygen to the cells, etc. When the culture material of the present invention is used, even plates having, for example, 1 well, 6 wells, 12 wells, 24 wells, 48 wells, 96 wells, 384 wells, or 1536 wells, have a stable shape and sufficiently supply oxygen to the cells, etc.

The culture tool is preferably an instrument having its bottom surface as the culture surface in order to hold or store the culture medium. If the culture tool is a dish, flask, insert, or plate, the bottom surface is the culture surface, and therefore of the bottom, side, and top surfaces thereof, the culture material of the present invention preferably constitutes at least part or all of the bottom surface. When at least the bottom surface (culture surface) is composed of the culture material of the present invention, oxygen can be supplied more efficiently to the culture medium through the culture material, and the cells, etc. present in the medium can be grown more efficiently. In addition, it is possible to culture at higher densities while retaining the functions of the cells.

The shape of the bottom surface of the culture tool is not particularly limited and examples thereof include a flat bottom, round bottom (U-bottom), flat bottom (F-bottom), conical bottom (V-bottom), and flat bottom with curved edge. If processing into, for example, a round bottom (U-bottom), flat bottom (F-bottom), conical bottom (V-bottom), or flat bottom with curved edge, the processing may be done at once by general injection molding or press molding, or it can also be produced by producing first a film or sheet and then performing secondary processing by vacuum molding or pressure molding, for example. The shape of the bottom surface is selected according to the purpose of culture, but a flat bottom is usually desirable when culturing the cells, etc. in two dimensions, and a round bottom (U-bottom) or conical bottom (V-bottom) is usually desirable when culturing in three dimensions.

The parts of the culture tool other than the culture material may be composed of a material other than the culture material. The material other than the culture material is not particularly limited, and a known material can be used. Examples of such material include polystyrene, polydimethylsiloxane (PDMS), cyclic olefin polymers, cyclic olefin copolymers, and glass.

The culture tool may be used after coating the culture surface thereof and/or the parts other than the culture surface with a natural polymer material, synthetic polymer material, or inorganic material.

When the culture surface of the culture tool is coated with a natural polymer material, synthetic polymer material, or inorganic material, the growth of the cells, etc. is improved, but the cells, etc. are more likely to adhere to the culture surface.

The presence or absence of a coating on the culture surface can be determined based on the type of cells, etc., but it is usually preferable not to coat.

The culture tool of the present invention may be disinfected or sterilized to prevent contamination. The method of disinfection or sterilization is not particularly limited, and examples thereof include physical disinfection methods such as free-flowing steam methods, boiling methods, intermittent methods, and ultraviolet methods; chemical disinfection methods using gases such as ozone or disinfectants such as ethanol; heat sterilization methods such as autoclaving methods and dry heat methods; irradiation sterilization methods such as gamma ray methods, electron beam methods, and high frequency methods; and gas sterilization methods such as ethylene oxide gas methods and hydrogen peroxide gas plasma methods. Of these, ethanol disinfection, autoclaved sterilization, gamma ray sterilization, electron beam sterilization, or ethylene oxide gas sterilization methods are preferred as they are easy to operate and achieve sufficient sterilization. These disinfection or sterilization treatments may be performed singly, or two or more may be performed in combination.

The method for producing the culture tool of the present invention is not particularly limited, and when the culture tool is entirely composed of the culture material, it can be produced by the same method as the method for producing the culture material. When a part of the culture tool is formed of the culture material, the culture tool can be obtained by appropriately joining the culture material and the other materials. The method for joining is not particularly limited, and the culture material and the other materials may be formed as a single piece, or they may be adhered to each other via an adhesive or pressure sensitive adhesive.

The culture tool of the present invention is preferably a culture tool for forming spheroids, and more preferably a cell culture tool for forming spheroids.

### [Culture Method]

The method for culturing cells, etc. of the present invention is a method for culturing cells, a tissue or an organ, the method including a step (A) of contacting the cells, tissue, or organ with the culture surface of a culture material, which is a culture material for culturing cells, a tissue, or an organ on a culture surface thereof, the culture material containing a 4-methyl-1-pentene polymer (X), having a water contact angle of the culture surface of more than 100° and 160° or less, and having an oxygen permeability at a temperature of 23°C and humidity of 0% of 4500 to 90000 cm³/(m²×24h×atm), or of a culture tool in which at least the culture surface is formed of the culture material; and a step (B) of culturing the cells, tissue, or organ in contact with the culture surface to form a spheroid.

The culture method of the present invention is one that allows cells, etc. to efficiently form spheroids when the cells, etc. are cultured on the culture surface of the culture material or the culture tool. In addition, according to the present invention, pluripotent stem cells more efficiently differentiate into differentiated cells.

### [Step (A)]

The method for contacting the cells, etc. with the culture surface of the culture material or culture tool is not particularly limited as long as it can contact the cells, etc. with the culture surface of the culture material or culture tool, and examples thereof include seeding the cells, etc. on the culture surface of the culture material or culture tool. More specifically, for example, the cells, etc. suspended in a culture medium are added to a culture vessel with, for example, a pipette, and the culture vessel is shaken as necessary to evenly distribute the cells in the culture vessel, which are then allowed to stand in an incubator.

The density at which the cells, etc. are seeded is not particularly limited as long as the cells, etc. can grow and differentiate. When the cells, etc. are undifferentiated pluripotent stem cells or differentiated cells derived from pluripotent stem cells, the seeding density is preferably 0.1 × 10⁵ cells/cm² to 10.0 × 10⁵ cells/cm², more preferably 0.3 × 10⁵ cells/cm² to 5.0 × 10⁵ cells/cm², and even more preferably 0.5 × 10⁵ cells/cm² to 3.0 × 10⁵ cells/cm².

A cell seeding density within the above range is preferred as the cell growth and differentiation can be performed more efficiently than when the cell seeding density is outside the above range.

The culture medium used for seeding is not particularly limited as long as it is a culture medium in which cells, etc. can survive, and may be appropriately selected according to the cells, etc. used. The culture medium used for seeding may be the culture medium used in the step (B) described below.

If the cells, etc. are undifferentiated pluripotent stem cells or differentiated cells derived from pluripotent stem cells, the culture medium used for seeding is, for example, any cell culture basal medium, differentiation medium, or medium dedicated to primary culture, and specific examples thereof include Essential 8, StemFit culture medium, ReproFF2 culture medium, Stem-Partner SF, Eagle's Minimum Essential Medium (EMEM), Dulbecco's Modified Eagle's Medium (DMEM), α-MEM, Glasgow's MEM (GMEM), IMDM, RPMI 1640, Ham's F-12, MCDB medium, William's medium E and mixed media thereof. Furthermore, a culture medium supplemented with, for example, serum, various growth factors, differentiation inducing factors, antibiotics, hormones, amino acids, sugars, salts, minerals, metals, or vitamins may be used.

The amount of culture medium used for seeding is not particularly limited, but the height of the culture medium is preferably 3 to 30 mm, more preferably 3 to 25 mm, and even more preferably 4 to 20 mm when added to the culture vessel.

The culture temperature is also not particularly limited, but the culture is usually performed at about 25 to 40°C.

### [Step (B)]

The method for culturing cells, etc. in contact with the culture surface to form spheroids is not particularly limited as long as oxygen and nutrients, for example, can be supplied to the cells, etc. in contact with the culture surface, and the cells, etc. can be cultured to form spheroids. Examples thereof include supplying oxygen to a culture incubator containing a culture tool to which a culture medium has been added, supplying oxygen to the cells, etc. via the culture material, and maintaining at 37°C for a certain period of time.

The culture medium used for cell culture may be appropriately selected according to the cells. The type of culture medium is not particularly limited, but for example, any cell culture basal medium, differentiation medium, or medium dedicated to primary culture can be used. Specific examples include Essential 8, Eagle's Minimum Essential Medium (EMEM), Dulbecco's Modified Eagle's Medium (DMEM), α-MEM, Glasgow's MEM (GMEM), IMDM, RPMI 1640, Ham's F-12, MCDB medium, William's medium E, Hepatocyte thaw medium, and mixed media thereof, but are not limited thereto, and any medium can be used as long as it contains the components necessary for cell growth and differentiation. Furthermore, a culture medium supplemented with, for example, serum, various growth factors, differentiation inducing factors, antibiotics, hormones, amino acids, sugars, or salts may be used. The culture temperature is also not particularly limited, but the culture is usually performed at about 25 to 40°C.

The amount of culture medium is not particularly limited, but the height of the culture medium is preferably 3 to 30 mm, more preferably 3 to 25 mm, and even more preferably 4 to 20 mm.

The culture period may be appropriately selected according to the type, size, and degree of differentiation of the cells and spheroids to be formed. When forming spheroids containing cardiomyocytes derived from iPS cells, the culture period is preferably 7 to 30 days, more preferably 10 to 25 days, and preferably 11 to 20 days.

### [Step (C)]

The method for culturing cells, etc. of the present invention may further include a step (C) of inducing differentiation of the cells, etc.

The method for inducing differentiation of the cells, etc. is not particularly limited. Induction of differentiation into the desired differentiated target cells may be performed according to a known protocol, and commercially available differentiation induction media or differentiation kits may be used.

The conditions for the induction of differentiation are not particularly limited and can be appropriately set according to, for example, the type of cell used and the type of differentiated target cell. Usually, differentiation can be induced by adding a predetermined cytokine, growth factor, or other compound at a predetermined concentration in the culture solution, and performing the culture.

When the method for culturing cells, etc. of the present invention includes the step (C), the cells, etc. are preferably undifferentiated pluripotent stem cells, differentiated cells derived from pluripotent stem cells (including pluripotent stem cells for which induction of differentiation has been initiated and which are already in the process of differentiation), undifferentiated somatic stem cells, or differentiated cells derived from somatic stem cells (including somatic stem cells for which induction of differentiation has been initiated and which are already in the process of differentiation), more preferably undifferentiated pluripotent stem cells or differentiated cells derived from pluripotent stem cells, and even more preferably undifferentiated iPS cells or differentiated cells derived from iPS cells.

The differentiated target cells are not particularly limited and may be any differentiated cells. The differentiated target cells include various progenitor cells, and are, for example, adipocytes, hepatocytes, renal cells, pancreatic cells, mammary gland cells, endothelial cells, epithelial cells, smooth muscle cells, myoblasts, cardiomyocytes, nerve cells, glial cells, dendritic cells, chondrocytes, osteoblasts, osteoclasts, osteocytes, fibroblasts, various blood cells, retinal cells, corneal cells, gonadal cells, or various glandular cells.

The differentiated target cells are preferably cells that form spheroids, and more preferably cells with a high oxygen demand. The differentiated target cells are preferably, for example, hepatocytes, nerve cells, cardiomyocytes, pancreatic β cells, vascular endothelial cells, adipocytes, adipose-derived stem cells, chondrocytes, mesenchymal stem cells, epithelial hair follicle stem cells, follicle dermal papilla cells, skin fibroblasts, skin keratinocytes, osteoblasts, and hematopoietic progenitor cells, and more preferably cardiomyocytes, nerve cells, and hepatocytes, and even more preferably cardiomyocytes.

The step (C) is preferably a step of inducing differentiation of pluripotent stem cells into cardiomyocytes, and more preferably a step of inducing differentiation of iPS cells into cardiomyocytes.

The method for inducing differentiation of pluripotent stem cells into cardiomyocytes is not particularly limited. As methods for inducing differentiation of pluripotent stem cells into cardiomyocytes, various approaches are known (e.g., Burridge et al., Cell Stem Cell. 2012 Jan 6;10(1):16-28; Kattman et al., Cell Stem Cell 2011; 8: 228-240; Zhang et al., Circ Res 2012; 111: 1125-1136; Lian et al., Nat Protoc 2013; 8: 162-175; WO 2016/076368; WO 2013/111875; and Minami et al., Cell Rep. 2012, 2(5): 1448-1460), and examples thereof include methods by embryoid body formation, methods by monolayer differentiation culture, and methods by forced aggregation. In any of these methods, the induction efficiency can be enhanced by sequentially allowing the action of, for example, mesoderm-inducing factors (e.g., activin A, BMP4, bFGF, VEGF, and SCF), cardiac determination factors (e.g., VEGF, DKK1, Wnt signaling inhibitors (e.g., IWR-1, IWP-2, and IWP-4), BMP signaling inhibitors (e.g., NOGGIN), TGFβ/activin/NODAL signaling inhibitors (e.g., SB431542), and retinoic acid signaling inhibitors), and cardiac differentiation factors (e.g., VEGF, bFGF, and DKK1).

The method for inducing differentiation of iPS cells into cardiomyocytes is not particularly limited, but for example, the known protein-free cardiac differentiation (PFCD) method can be used (see WO2015/182765). Since the protein-free cardiac differentiation (PFCD) method can achieve a high efficiency of cardiac differentiation, the step (C) is preferably a step of inducing differentiation of iPS cells into cardiomyocytes by the protein-free cardiac differentiation (PFCD) method.

The step (C) may be performed after the step (A) and before the step (B), at the same time as the step (B), or only during part of the period in which the step (B) is performed. The step (C) is preferably performed at the same time as the step (B), as it allows to more efficiently induce the differentiation of pluripotent stem cells.

The period of induction of differentiation in the step (C) can be appropriately set according to, for example, the type of cell used, the type of differentiated target cell, the degree of differentiation, and the method for inducing differentiation. When inducing differentiation of iPS cells into cardiomyocytes, the period of induction of differentiation is preferably 7 to 30 days, more preferably 10 to 25 days, and even more preferably 11 to 20 days.

### [Characteristics of Culture Method]

The method for culturing cells, etc. of the present invention is preferably a method for culturing iPS cells or differentiated cells derived from iPS cells, and more preferably a method for culturing cardiomyocytes derived from iPS cells.

The method for culturing cells, etc. of the present invention is preferably one that increases cardiac purity.

The above increase refers to when, using as an object of comparison the cardiac purity obtained when pluripotent stem cells are differentiated into cardiomyocytes under experimental conditions in which a polystyrene culture material or culture tool is used instead of the above culture material or culture tool and the other conditions are the same as those of the method for culturing cells, etc. of the present invention, the cardiac purity is higher than that of the object of comparison. The cardiac purity is preferably 1.3 times or more, more preferably 2 times or more, and even more preferably 3 times or more higher than that of the above object of comparison.

The cardiac purity is the ratio (%) of the number of cardiomyocytes differentiated from pluripotent stem cells to the total number of differentiated cells derived from pluripotent stem cells. The method for measuring the cardiac purity is not particularly limited, but for example, the ratio can be calculated by measuring the total number of cells and the number of cells expressing a cardiomyocyte marker after a predetermined period of induction of differentiation. If the cells are forming a spheroid, the spheroid can be dissociated into single cells by a known method and the total number of cells constituting the spheroid can be used as the total number of cells.

For cell counting, for example, a hemocytometer or FACS can be used.

The cardiomyocyte marker is not particularly limited, and examples thereof include cardiac troponin C (cTnC), cardiac troponin I (cTnI), cardiac troponin T (cTnT), myosin light chain, α-actinin, NKX2.5, KCNQ1, HERG1b, Cav1.2, and Nav1.5. The cardiomyocyte marker is preferably cardiac troponin T (cTnT).

The number of cells expressing a cardiomyocyte marker can be measured, for example, by FACS using antibodies against the cardiomyocyte marker.

The cardiac purity can preferably be calculated by the number of cTnT-positive cells/total number of cells constituting the spheroid × 100.

The method for culturing cells, etc. of the present invention is preferably one that increases the expression of the MYL2 (Myosin regulatory light chain 2) gene in cardiomyocytes.

The above increase refers to when, using as an object of comparison the expression level of the MYL2 gene in cardiomyocytes when pluripotent stem cells are differentiated into cardiomyocytes under experimental conditions in which a polystyrene culture material or culture tool is used instead of the above culture material or culture tool and the other conditions are the same as those of the method for culturing cells, etc. of the present invention, the expression level of the MYL2 gene is higher than that of the object of comparison. The expression level of the MYL2 gene is preferably 2 times or more, and more preferably 3 times or more higher than that of the above object of comparison.

The expression level of the MYL2 gene can be measured by a known method, and examples thereof include the quantitative RT-PCR method.

The method for culturing cells, etc. of the present invention is preferably one that increases the beating rate of cardiomyocytes.

The above increase refers to when, using as an object of comparison the beating rate of cardiomyocytes when pluripotent stem cells are differentiated into cardiomyocytes under experimental conditions in which a polystyrene culture material or culture tool is used instead of the above culture material or culture tool and the other conditions are the same as those of the method for culturing cells, etc. of the present invention, the beating rate is higher than that of the object of comparison. The beating rate of the cardiomyocytes is preferably 2 times or more, and more preferably 3 times or more higher than that of the above object of comparison.

The beating rate of cardiomyocytes can be measured by a known method, and for example, the BPM (number of beats per minute) of any cardiomyocyte can be measured by taking a video of the cardiomyocyte.

### [Spheroid]

The spheroid of the present invention is formed by the above culture method.

The spheroid of the present invention is characterized by its tendency to be highly uniform in size and shape because the oxygen is supplied to the inside of the cells during culture. In addition, the cells contained in the spheroid of the present invention possess normal function and their cell purity is also high, which increases the reliability and reproducibility of the data when the spheroid is used for various tests.

The spheroid of the present invention can be suitably used for the evaluation of cell function and drug screening, as well as a source of cells for regenerative medicine and cell therapy.

### EXAMPLES

Next, the present invention will be described in more detail with reference to Examples, but the present invention is not limited to thereto.

### [Measurement of Weight Average Molecular Weight (Mw) and Molecular Weight Distribution (Mw/Mn)]

The weight average molecular weight Mw and molecular weight distribution (Mw/Mn) of the 4-methyl-1-pentene polymer used in the Examples were measured by gel permeation chromatography (GPC).

Specifically, the weight average molecular weight (Mw) and number average molecular weight (Mn) of the polymer dissolved in o-dichlorobenzene were measured under the following conditions, with the molecular weight calibrated by standard polystyrene.
- Device: Gel Permeation Chromatograph HLC-8321 GPC/HT (manufactured by Tosoh Corporation)
- Data analysis software: Empowers (manufactured by Waters)
- Detector: Differential refractometer
- Columns connected in series: TSKgel GMH6-HT (2 columns) and TSKgel GMH6-HTL (2 columns)
- Column temperature: 140°C
- Flow rate: 1.0 ml/min
- Sample concentration: 1.5 mg/mL

### [Measurement of Sagging Distance]

A test piece was cut from the film of the Production Example or the culture vessel of the Comparative Example to a size of 100 mm × 10 mm in length × width, and fixed onto a test board in a state where the test piece protrudes lengthwise a length of 50 mm in a horizontal direction from a top surface of the test board, the top surface being horizontal . Then, 3 minutes after the fixing, a measurement is performed of a distance of how much an end of the test piece protruding from the test board sags in a vertically downward direction from a horizontal plane including the top surface of the test board. The room temperature was 23°C from the fixing to the measurement. The results are shown in Table 1.

### [Presence of warping]

On day 19 of induction of differentiation, the culture vessel was removed from the incubator the bottom surface of each vessel was viewed laterally, and the presence or absence of sagging of the film in the culture environment was observed. When there was no change compared to when the container was produced and no sagging of the film was observed, it was evaluated as "no warping," and when there was a change compared to when the culture container was produced and sagging of the film was observed, it was evaluated as "warping."

### [Measurement of Water Contact Angle]

The water contact angle was measured in accordance with the Japanese Industrial Standard JIS-R3257 (Testing method of wettability of glass substrate). Under constant temperature and humidity conditions of 25 ± 5°C and 50 ± 10%, a drop of water with a volume of 4 µL or less, the shape of which can be considered as spherical, was added dropwise on the surface of a measurement sample, and then the angle of the contact interface between the measurement sample and the drop of water within one minute immediately after the drop of water makes contact with the surface of the measurement sample was measured by the sessile drop method.

### [Measurement of Oxygen Permeability]

The oxygen permeability coefficient of a measurement sample was measured using the differential pressure gas permeability measuring device MT-C3 manufactured by Toyo Seiki Seisaku-sho, Ltd. under an environment of temperature of 23°C and humidity of 0%. The diameter of the measurement section was 70 mm (permeation area: 38.46 cm²). As the oxygen permeability coefficient was expected to be large, the sample was covered with an aluminum mask beforehand to have an actual permeation area of 5.0 cm².

The value of the measured oxygen permeability coefficient [cm³×mm/ (m²×24h×atm)] was divided by the thickness (µm) of the film (culture material) to calculate the oxygen permeability [cm³/ (m²×24h×atm)] .

### [Production Example 1] Production of Culture Material

TPX^{™}, a 4-methyl-1-pentene polymer (manufactured by Mitsui Chemicals, Inc.: molecular weight (Mw) = 428000, molecular weight distribution (Mw/Mn) = 4.1) was used, charged into an extruder with T-die equipped with a full-flight screw that extrudes the base layer, and extruded while setting the extrusion temperature at 270°C and the roll temperature at 60°C, and varying the conditions of roll rotation speed to obtain a 50 um thick film. The water contact angle and oxygen permeability were measured for the above film obtained. The results are shown in Table 1.

### [Production Example 2] Production of Culture Vessel

The above film was cut to 8 cm × 12 cm size and adhered to the bottom of a polystyrene (also referred to as PS) 24-well container frame via a medical adhesive (manufactured by 3M) to produce a 24-well culture plate. The culture plate was then packaged in a gamma-ray resistant bag and sterilized by irradiation with gamma rays of 10 kGy.

### [Example 1] Induction of Differentiation of iPS Cells into Cardiomyocytes

Using the culture vessel produced in Production Example 2, differentiation of iPS cells into cardiomyocytes was induced by the method described below, and spheroids were formed. The culture medium was used so as to achieve a culture medium height of 5 mm (culture medium volume: 1 mL).

### [Example 2]

The culture was performed in the same manner as in Example 1, except that the amount of culture medium was increased compared to Example 1 and the height of the culture medium was 10 mm (culture medium volume: 1.8 mL).

### [Comparative Example 1]

The culture was performed in the same manner as in Example 1, except that an Ultra-Low Attachment PS culture vessel (Costar^{™} 3473 manufactured by Corning Incorporated, inner diameter of culture surface of one well: 15 mm, 24-well plate) with a culture surface thickness of 1000 um was used. The Ultra-Low Attachment PS culture vessel is a commercially available PS culture vessel (water contact angle: 61°) that has been made hydrophilic. The results of measuring the water contact angle are shown in Table 1.

### [Comparative Example 2]

The culture was performed in the same manner as in Comparative Example 1, except that the amount of culture medium was increased compared to Comparative Example 1 and the height of the culture medium was 10 mm.

### [iPS Cell Culture]

### <Preparation of culture medium and reagents>

Human-derived iPS cells (provided by the Center for iPS Cell Research and Application) cryopreserved in liquid nitrogen were used.

The reagents used are as follows.
- Essential 8 (Product No. A1517001, Gibco)
- iMatrix-511 silk (Product No. 387-10131, Nippi. Inc.)
- CultureSure^{™} Y-27632 (Product No. 034-24024, FUJIFILM Wako Pure Chemical Corporation)
- 0.5 mol/L-EDTA solution (pH 8.0) (Product No. 06894-85, NACALAI TESQUE, INC.)
- PBS (-) (Product No. 166-23555, FUJIFILM Wako Pure Chemical Corporation)
- 2.5% Trypsin (Product No. 15090-046, Gibco)
- Trypan blue (Product No. 145-0022, Bio-Rad Laboratories, Inc. )
- Formaldehyde solution (Product No. 064-00406, FUJIFILM Wako Pure Chemical Corporation)
- anti-Troponin T-C (CT3) (Product No. SC-20025, Santa Cruz Biotechnology, Inc.)
- anti-Mouse IgG (H+L) Alexa Fluor 647 (Product No. A-21236, Thermo Fisher Scientific)
- Saponin from Soybeans (Product No. 192-08851, FUJIFILM Wako Pure Chemical Corporation)
- Medium for inducing cardiac differentiation (Low molecular compound group A, low molecular compound group B): according to the description in WO2015/182765, paragraphs [0064] and [0065].

The culture medium and reagents were prepared as follows.
- iPS cell culture medium: at the time of use, Essential 8 was brought to room temperature before use. The culture medium used during thawing and passaging was supplemented with CultureSure^{™} Y-27632 at 3 µM before use.
- Medium for inducing cardiac differentiation: at the time of use, the medium for inducing cardiac differentiation was mixed with the low molecular compound group A or low molecular compound group B and heated to 37°C before use.
- Dissociation solution: 0.5 mol/L-EDTA solution (pH 8.0) was adjusted to 0.5 mM with PBS (-). At the time of use, it was heated to 37°C before use.
- Y-27632 solution: was adjusted to 10 mM with PBS (-).
- Single-cell dissociation solution: 2.5% Trypsin was adjusted to 0.25% with PBS (-). At the time of use, it was heated to 37°C before use.
- Saponin solution: Saponin from Soybeans was adjusted to a 0.1% solution with PBS (-).
- Storage conditions: the Y-27632 solution, single-cell dissociation solution, and other reagents necessary for cardiomyocyte culture were stored in a freezer, and the culture medium and dissociation solution were stored under refrigeration.

### <Culture Schedule>

The cell culture was performed according to the following schedule. iPS cells passaged 5 times after revival were induced to differentiate. Sampling was performed on day 12, when the beating of the cardiomyocytes was generally confirmed, and on day 19, when the cardiomyocytes were more mature.
Day -1: Preparation of reagents and the like
Day 0: Revival of iPS cells
From day 3: iPS cell passaging (days 3, 6, 10, 15, and 19, total: 5 passages)
Day 23: Initiation of induction of cardiac differentiation of iPS cells (Day 0)
Day 45: Sampling on day 12 of induction of cardiac differentiation and dissociation into single cells (FACS, gene expression analysis)
Day 39: Sampling on day 19 of induction of cardiac differentiation and dissociation into single cells (FACS, gene expression analysis)

### <Method for culturing iPS cells>

### Thawing of iPS cells

1) Before thawing the iPS cells, 10 mL of culture medium was placed in tubes and heated in a 37°C water bath.
2) Frozen iPS cell tubes were half thawed in a 37°C water bath and slowly transferred to the preheated tubes containing 10 mL of culture medium in a clean bench.
3) After centrifugation at 100 × g for 3 minutes at room temperature, the supernatant was removed.
4) 10 mL of new culture medium was added and lightly mixed, then after adding 0.13 µg/cm² of iMatrix-511 silk to the cell suspension, the cells were seeded in 10 cm dishes and cultured in a 37°C, 5% CO₂ incubator.

### Passaging of iPS cells

1) The condition of the cells was observed with a phase contrast microscope and the split ratio for passaging was determined between 1:12-1:20.
2) The medium supernatant was collected in tubes. After washing with PBS (-), the dissociation solution was added and allowed to react in a 37°C, 5% CO₂ incubator for about 5 minutes.
3) After the reaction, the dish was tilted to detach the cells and the supernatant previously collected was used to collect the cells in tubes.
4) After centrifugation at 100 × g for 3 minutes at room temperature, the supernatant was removed.
5) 10 mL of new culture medium was added and lightly mixed, then after adding 0.13 µg/cm² of iMatrix-511 silk to the cell suspension, the cells were seeded in 10 cm dishes and cultured in a 37°C, 5% CO₂ incubator.

### <Method for inducing cardiac differentiation>

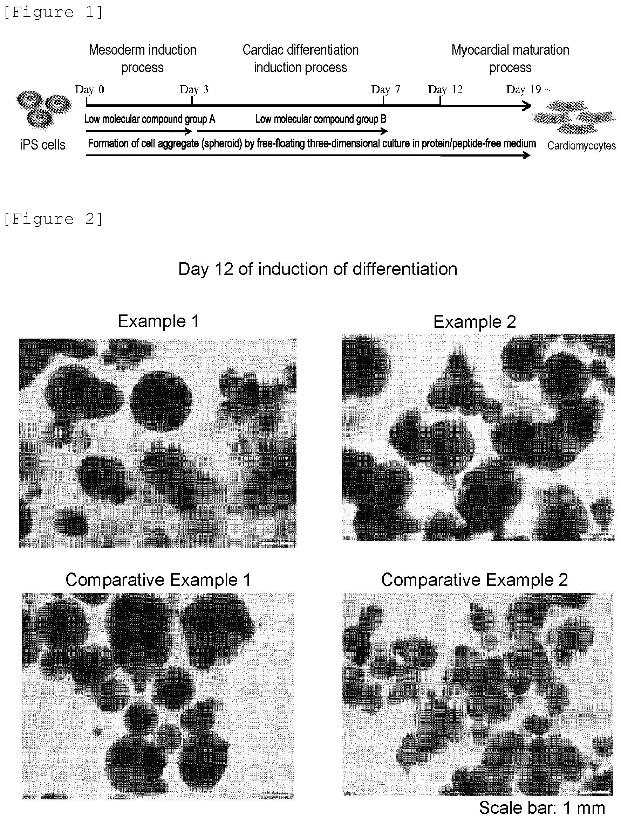

An outline of the experimental schedule is shown in Figure 1. Differentiation of iPS cells into cardiomyocytes was induced by the protein-free cardiac differentiation (PFCD) method.
Day 0: About 50% confluent iPS cells were detached with the dissociation solution and centrifuged at 100 × g for 3 minutes at room temperature. After removal of the supernatant, the cells were suspended in new medium, seeded in an untreated 55 cm² dish, and allowed to stand in a 37°C, 5% CO₂ incubator for 4 hours. The cells were then collected in tubes and the medium replaced with medium for inducing cardiac differentiation supplemented with low molecular compound group A. The cells were seeded at 2 × 10⁵ cells/well in the 24-well plates of the Examples or Comparative Examples and cultured in a 37°C, 5% CO₂ incubator. The experiments were performed in triplicate.
Day 3: The medium was replaced with medium for inducing cardiac differentiation containing low molecular compound group B.
Day 5: The medium was replaced with medium for inducing cardiac differentiation containing low molecular compound group B.
Day 7 onward: the medium was replaced every 3 to 4 days with medium for inducing cardiac differentiation. The wells were replaced when the medium was replaced on days 3 and 7 of induction of differentiation.

### <Dissociation of cell mass into single cells (sampling on days 12 and 19 of induction of differentiation)>

1) Cell masses (thought to be myocardial masses) were collected in a tube and the supernatant was removed once the cell masses had spontaneously settled.
2) 1 mL of the single-cell dissociation solution was added, and the mixture was heated in a 37°C water bath for around 30 minutes, and agitated until the cell masses dissociated into single cells.
3) The reaction was stopped by diluting with 3 mL of 3x PBS (-).
4) Cell counting of the cell suspension was performed and used for FACS or RNA extraction as described below.

### <Preparation of FACS sample>

1 × 10⁶ cells dissociated into single cells were transferred to a tube, and after centrifugation at 300 × g for 5 minutes at room temperature, the supernatant was removed and the cells were resuspended in 1mL of PBS (-). Formaldehyde (FUJIFILM Wako Pure Chemical Corporation) was added to the cell suspension to a final concentration of 4% and allowed to stand for 5 minutes at room temperature. After centrifuging at 300 × g for 3 minutes at room temperature and removing the supernatant, 1 mL of saponin solution was added to suspend the cells, of which 0.7 mL (sample) and 0.3 mL (negative control) were dispensed respectively into two tubes. After centrifuging again and removing the supernatant, 0.5 mL of saponin solution with anti-Troponin T-C (CT3) antibody added at 1:1000 as a primary antibody was added to the sample. For the negative control, the cells were suspended by adding 0.5 mL of saponin solution without antibody. The antibody was allowed to react overnight at 4°C. The following day, the primary antibody was removed and 0.5 mL of saponin solution with anti-Mouse IgG (H+L) Alexa Fluor 647 added at 1:500 as a secondary antibody was added. The sample was shielded from light, and the antibody was allowed to react for 1 hour at room temperature. After 1 hour, the saponin solution with the secondary antibody was replaced with 0.5 mL of PBS (-) and analyzed by FACS (Accuri^{™} CS6 Plus (manufactured by BD)).

### <RNA extraction and RT-qPCR>

Troponin (gene specific to cardiomyocytes), MYL2 (gene specific to ventricular muscle, which are cardiomyocytes), and MYL7 (gene specific to atrial muscle, which are cardiomyocytes) were analyzed.

### (1) Reagents and equipment RNA extraction: miRNeasy Mini Kit (Product No. 217004, manufactured by QIAGEN)

cDNA synthesis: ReverTra Ace (R) qPCR RT Master Mix with gDNA Remover (Product No. FSQ-301, manufactured by TOYOBO CO.LTD.) qPCR reaction: PowerUp SYBR Green Master Mix (Product No. A25776, manufactured by Thermo Fisher Scientific)
QuantStudio 6 Flex Real-time PCR system (manufactured by Thermo Fisher Scientific)
Nanophotometer Spectrophotometer C40 (manufactured by WakenBtech Co., Ltd.)

### (2) RNA extraction

After removing the culture supernatant on days 12 and 19 of induction of differentiation into cardiomyocytes, 0.5 ml of QIAZOL (manufactured by QIAGEN) was added and suspended to lyse the cells, and the lysate was collected in a 1.5 ml tube. From there on, RNA was extracted according to the protocol provided with the miRNeasy Mini Kit, and the RNA concentration was measured with the Nanophotometer C40.

### (3) RT-qPCR

Using 1 µg of the RNA extracted above, a reverse transcription reaction was performed to synthesize cDNA according to the protocol provided with the PowerUp SYBR Green Master Mix. The qPCR reaction was then performed by the standard method using 6 ng of cDNA. The calibration curves were prepared by collecting 10 µL of each cDNA sample at 10 ng/uL, and diluting them to 1/10 to obtain 5 points.

### <Evaluation of cardiac-specific gene expression>

RNA extracted from cells on days 12 and 19 of induction of differentiation was used to analyze gene expression levels with the QuantStudio 6 Flex Real-time PCR system. The primer sequences used are shown in Table 2 and the PCR conditions in Table 3. The gene expression levels are expressed as values relative to a gene expression level of glyceraldehyde-3-phosphate dehydrogenase (GAPDH) of 1. The results are shown in Table 4.

### <Evaluation of cell count>

After dissociating the cell masses at days 12 and 19 of induction of differentiation into single cells, live cells were counted by trypan blue staining (Bio-Rad Laboratories, Inc.) (using a TC20 automated cell counter, Bio-Rad Laboratories, Inc.).

From the measured values obtained above, the cell viability (%) (number of live cells at the time of measurement/total number of cells at the time of measurement × 100) and cell proliferation rate (%) (number of live cells at the time of measurement/number of cells at the time of cell seeding (start of cell culture) × 100) were calculated. The results are shown in Table 4.

### <Evaluation of cardiac purity>

After dissociating the cell masses at days 12 and 19 of induction of differentiation into single cells, the cTnT-positive rate (number of cTnT-positive cells/total number of cells constituting the cell mass × 100) was calculated by FACS and used as the cardiac purity (%). The results are shown in Table 4.

### <Evaluation of beating rate>

A video of the cells on day 19 of induction of differentiation was taken and the BPM (number of beats per minute, beats/minute) of an arbitrary spheroid was calculated. The results are shown in Table 4.

**[Table 1]**

| | Example 1 | Example 2 | Comparative Example 1 | Comparative Example 2 |
|---|---|---|---|---|
| Material of vessel bottom | TPX | TPX | TCPS | TCPS |
| Mw×10000 (Mw/Mn) | 42.8 (4.1) | 42.8 (4.1) | | |
| Sagging distance (mm) | 0 | 0 | 0 | 0 |
| Presence of warping | No | No | No | No |
| Water contact angle (°) | 110 | 110 | 40 | 40 |
| Thickness of vessel bottom (mm) | 0.05 | 0.05 | 1 | 1 |
| Oxygen permeability coefficient of vessel bottom (cm^{3∗}mm/m^{2∗}24h^{∗}atm) | 1912 | 1912 | 203 | 203 |
| Oxygen permeability of vessel bottom (cm³/m^{2∗}24h^{∗}atm) | 38240 | 38240 | 203 | 203 |
| Culture medium height (mm) | 5 | 10 | 5 | 10 |

**[Table 2]**

| Gene name | Direction | Sequence (5'-3') | Sequence No. |
|---|---|---|---|
| GAPDH | Fw | ATTGCCCTCAACGACCACTTTG | 1 |
| | Rv | TCTTCCTCTTGTGCTCTTGCTG | 2 |
| MYL2 | Fw | AGGCTCCGGGTCCAATTAAC | 3 |
| | Rv | TTGCCTTCAGGGTCAAACAC | 4 |
| MYL7 | Fw | TCAGCTGTATCGACCAGAATCG | 5 |
| | Rv | AGGAAGACGGTGAAGTTGATGG | 6 |

**[Table 3]**

| Step | Temperature | Duration | No. of cycles |
|---|---|---|---|
| UDG activation | 50°C | 2 min | Hold |
| Dual Lock DNA Polymerase | 95°C | 2 min | Hold |
| Modification | 95°C | 15 s | 40 |
| Annealing/Elongation | 60°C | 1 min | |

**[Table 4]**

| | Example 1 | Example 2 | Comparative Example 1 | Comparative Example 2 |
|---|---|---|---|---|
| Cell viability (%) at day 12 of induction of differentiation | 69.0 | 63.5 | 80.3 | 74.1 |
| Cell proliferation rate (%) at day 12 of induction of differentiation | 1000 | 1250 | 270 | 650 |
| Cardiac purity (%) at day 12 of induction of differentiation | 15.3 | 53.2 | 1.4 | 29.9 |
| Troponin gene expression level at day 12 of induction of differentiation | 2.7 | 5.5 | 0.3 | 2.2 |
| MYL2 gene expression level at day 12 of induction of differentiation | 0.15 | 0.48 | 0.02 | 0.12 |
| MYL7 gene expression level at day 12 of induction of differentiation | 9.4 | 18.1 | 0.73 | 6.5 |
| Cell viability (%) at day 19 of induction of differentiation | 73.5 | 69.1 | 78.8 | 67.8 |
| Cell proliferation rate (%) at day 19 of induction of differentiation | 1430 | 2790 | 540 | 820 |
| Cardiac purity (%) at day 19 of induction of differentiation | 13.2 | 42.6 | 2.4 | 16.5 |
| Troponin gene expression level at day 19 of induction of differentiation | 1.5 | 4.0 | 0.6 | 1.1 |
| MYL2 gene expression level at day 19 of induction of differentiation | 0.72 | 8.50 | 0.15 | 0.34 |
| MYL7 gene expression level at day 19 of induction of differentiation | 1.50 | 3.90 | 0.26 | 0.80 |
| Myocardial beating rate BPM (beats/min) | 59.8 | 61.6 | 18 | 43.6 |

### <Morphological observation>

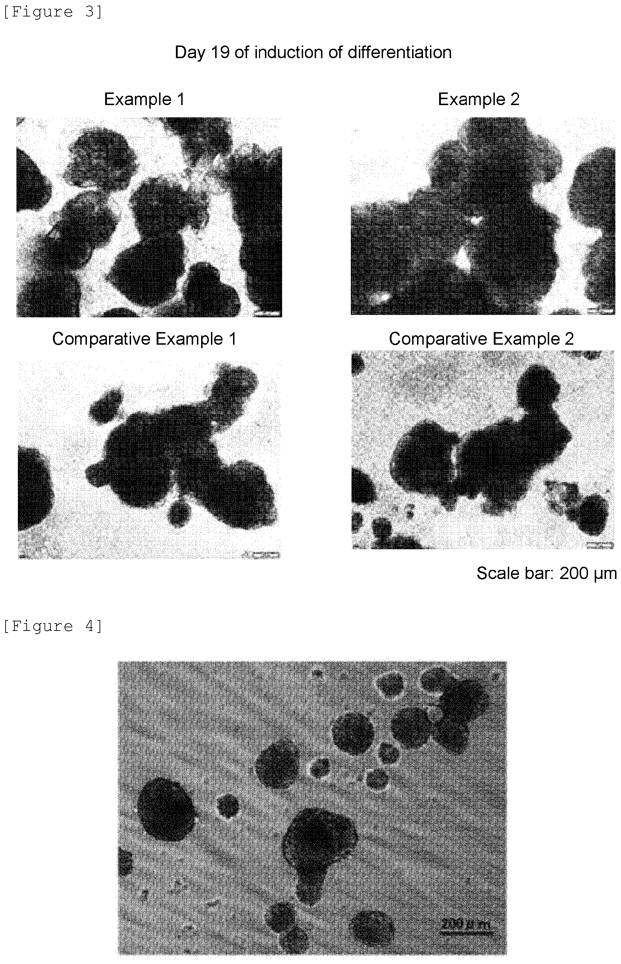

A phase contrast microscope was used to observe the formation of each cell into spheroids. The results are shown in Figures 2 and 3.

### <Results>

On day 12 of induction of differentiation, the cell proliferation rate was higher in Examples 1 and 2 than in Comparative Examples 1 and 2 due to a higher oxygen permeability of the bottom of the culture, and was particularly higher at a culture medium height of 10 mm. A similar trend was observed on day 19. In addition, in Examples 1 and 2, the cardiac purity was higher than in Comparative Examples 1 and 2 on days 12 and 19 of induction of differentiation. Furthermore, when comparing the expression of the cardiac-specific genes Troponin, MYL2, and MYL7, all genes had higher levels of expression in Examples 1 and 2 than in Comparative Examples 1 and 2, and particularly at a culture medium height of 10 mm. In Examples 1 and 2, the beating rate was higher than in Comparative Examples 1 and 2 on day 19 of induction of differentiation, and the beating rate was higher at a liquid depth of 10 mm than 5 mm. The results of morphological observation revealed that in Examples 1 and 2, a plurality of spheroids with uniform size and shape were formed compared to Comparative Examples 1 and 2. It was also found that the iPS cells differentiated into cardiomyocytes more efficiently in Examples 1 and 2 than in Comparative Examples 1 and 2.

### [Example 3] Culture of Human Osteosarcoma-derived Cancer Cells (HOF-143B)

Using the culture vessel produced in Production Example 2, human osteosarcoma-derived cancer cells (HOF-143B) were cultured by the method described below, and spheroids were formed.

### [Comparative Example 3]

The culture was performed in the same manner as in Example 3, except that a commercially available TCPS culture vessel (inner diameter of culture surface: 16 mm, 24-well plate, manufactured by Corning Incorporated, polystyrene (PS)) with a culture surface thickness of 1000 um was used.

### [Comparative Example 4]

The culture was performed in the same manner as in Example 3, except that a commercially available PDMS (polydimethylsiloxane) culture vessel (inner diameter of culture surface: 15 mm, 24-well plate, product name: G-plate, manufactured by Vessel Inc., model No. V24WGPB) with a culture material thickness of 350 um was used as a highly oxygen permeable vessel.

### [Culture of human osteosarcoma-derived cancer cells (HOF-143B)]

### <Seeding of cells>

A culture medium was added to a centrifuge tube (50 ml) containing a cell suspension containing human osteosarcoma-derived cancer cells. The culture medium was prepared by adding 5 mL of fetal bovine serum (FBS, FUJIFILM Wako Pure Chemical Corporation), 0.5 mL of 200 mM L-glutamine solution (FUJIFILM Wako Pure Chemical Corporation), 0.05 mL of 1.5 mg/mL Bromo-deoxy Uridine (BUdR), 0.5 mL of Non Essential Amino Acid (FUJIFILM Wako Pure Chemical Corporation), and 43.95 mL of E-MEM medium (containing 10 mg/mL phenol red and 2200 mg/mL sodium bicarbonate, for culture, FUJIFILM Wako Pure Chemical Corporation). Cell density was adjusted by a method of adjusting the number of cells in the cell suspension containing the human osteosarcoma-derived cancer cells, and the cell suspension was prepared to obtain a cell density of 1.0 × 10⁴ cells/cm² when seeding at 0.5 mL/well of cell suspension.

### <Culture of cells>

The cell suspension containing the human osteosarcoma-derived cancer cells was seeded on the culture surface with a micropipette and brought into an incubator to initiate culture at 37°C and 5% CO₂. The culture was performed for 7 days. The culture medium was replaced on days 3 and 6 after cell seeding. The medium replacement was performed by allowing the culture vessel to stand still, removing the supernatant when the spheroids had sufficiently settled, and adding the amount of medium that had been removed.

### <Morphological observation and evaluation>

Cell morphology was observed using a phase contrast microscope on day 7 of culture and evaluated according to the following criteria.
A: Spheroids are formed
B: Cells are adhering and not forming spheroids

The results of observing Example 3 are shown in Figure 4. The results of evaluation are shown in Table 5.

**[Table 5]**

| | Example 3 | Comparative Example 3 | Comparative Example 4 |
|---|---|---|---|
| Material of vessel bottom | TPX | TCPS | PDMS |
| Thickness of vessel bottom (mm) | 0.05 | 1 | 0.35 |
| Morphology evaluation | A | B | B |

### <Results>

In Comparative Examples 3 and 4, the cells adhered to the culture vessel and did not form spheroids, whereas in Example 3, the cells formed spheroids.

### Industrial Applicability

These results clearly show that spheroids of cells retaining normal function are formed when the culture material of the present invention is used. They also show that iPS cells efficiently differentiates into cardiomyocytes when the culture material of the present invention is used. That is, the culture material of the present invention facilitates the formation of spheroids and the differentiation of pluripotent stem cells. Therefore, the culture material of the present invention can be suitably used in drug discovery screening and diagnostic applications, as well as in regenerative medicine applications.

## Claims

1. A culture material for culturing cells, a tissue, or an organ on a culture surface thereof, the culture material comprising a 4-methyl-1-pentene polymer (X), having a water contact angle of the culture surface of more than 100° and 160° or less, and having an oxygen permeability at a temperature of 23°C and humidity of 0% of 4500 to 90000 cm³/ (m²×24h×atm).

2. The culture material according to claim 1, wherein the 4-methyl-1-pentene polymer (X) is a copolymer (x1) of 4-methyl-1-pentene and at least one selected from ethylene and α-olefins having 3 to 20 carbon atoms (excluding 4-methyl-1-pentene) .

3. The culture material according to claim 1 or 2, wherein the culture surface is not processed to form an uneven structure.

4. The culture material according to any one of claims 1 to 3, which is for spheroid formation.

5. The culture material according to any one of claims 1 to 4, wherein the cells, tissue, or organ comprise differentiated cells derived from iPS cells.

6. The culture material according to any one of claims 1 to 5, wherein the cells, tissue, or organ comprise cardiomyocytes.

7. The culture material according to any one of claims 1 to 6, wherein the shape of the culture material is in the form of a film or a sheet.

8. A culture tool comprising at least a culture surface formed of the culture material according to any one of claims 1 to 7.

9. A method for culturing cells, a tissue, or an organ, the method comprising:
a step (A) of contacting the cells, tissue, or organ with the culture surface of the culture material according to any one of claims 1 to 7 or the culture tool according to claim 8; and
a step (B) of culturing the cells, tissue, or organ in contact with the culture surface to form a spheroid.

10. The method for culturing cells, a tissue, or an organ according to claim 9, further comprising a step (C) of inducing differentiation of, for example, the cells, wherein the step (C) is a step of inducing differentiation of iPS cells into cardiomyocytes by the protein-free cardiac differentiation (PFCD) method.

11. The method for culturing cells, a tissue or an organ according to claim 9 or 10 that increases cardiac purity.

12. A spheroid formed by the culture method according to any one of claims 9 to 11.
